Europäisches Patentamt

European Patent Office

Office européen des brevets

(19)

(11) Veröffentlichungsnummer : **0 040 709**
**B1**

(12) **EUROPÄISCHE PATENTSCHRIFT**

(45) Veröffentlichungstag der Patentschrift :
04.07.84

(21) Anmeldenummer : 81103219.2

(22) Anmeldetag : 29.04.81

(51) Int. Cl.³ : **C 07 D307/62, C 07 D493/14, C 25 B 3/02, B 01 D 13/02**

(54) **Verfahren zur Herstellung von Diacetonketogulonsäure.**

(30) Priorität : 27.05.80 DE 3020104

(43) Veröffentlichungstag der Anmeldung :
02.12.81 Patentblatt 81/48

(45) Bekanntmachung des Hinweises auf die Patenterteilung : 04.07.84 Patentblatt 84/27

(84) Benannte Vertragsstaaten :
CH DE FR GB LI

(56) Entgegenhaltungen :
DE-A- 2 045 723
DE-B- 2 410 034
US-A- 2 559 034

(73) Patentinhaber : **Merck Patent Gesellschaft mit beschränkter Haftung**
**Frankfurter Strasse 250**
**D-6100 Darmstadt (DE)**

(72) Erfinder : **Reiff, Fritz, Dr.**
**Im Rassdorf 6**
**D-6104 Seeheim-Jugenheim 1 (DE)**
Erfinder : **Wintermeyer, Willi, Dr.**
**Gartenstrasse 4**
**D-6104 Seeheim-Jugenheim 2 (DE)**
Erfinder : **Wittmann, Rolf, Dr.**
**Tannenstrasse 6**
**D-6109 Mühltal-Traisa (DE)**
Erfinder : **Butzke, Jürgen**
**Waldstrasse 48**
**D-6110 Dieburg (DE)**
Erfinder : **Müller, Peter**
**Kleiststrasse 14**
**D-6100 Darmstadt-Arheilgen (DE)**

Jouve, 18, rue St-Denis, 75001 Paris, France

## Beschreibung

Die Erfindung betrifft ein Verfahren zur Herstellung von Diacetonketogulonsäure, die ein wertvolles Zwischenprodukt bei der Herstellung von Vitamin C darstellt.

Bei der Herstellung von Diacetonketogulonsäure geht man in der Regel von Diacetonsorbose aus, die z. B. mit anorganischen Oxidationsmitteln wie $HNO_3$, $H_2O_2$ oder Hypochlorit zu Diacetonketogulonsäure oxidiert werden kann. Besonderes Interesse haben in letzter Zeit sowohl die katalysierte Oxidation mit Luft bzw. sauerstoffhaltigen Gasen als auch die elektrochemische Oxidation gefunden. Bei diesen Verfahren werden zu Beginn der Reaktion sehr gute Umsätze erzielt, die Reaktion verlangsamt sich jedoch zunehmend mit steigendem Gehalt des Oxidationsproduktes. Um eine vollständige Umsetzung der diacetonsorbose zu erzielen, müssen relativ lange Reaktionszeiten unter verschärften Bedingungen in Kauf genommen werden, wobei die Gefahr einer Weiteroxidation der gebildeten Diacetonketogulonsäure besteht, die zu einer Ausbeuteminderung und unerwünschten Nebenprodukten führt.

Es bestand deshalb die Aufgabe, ein Verfahren zu finden, nach dem Diacetonketogulonsäure mit guten Umsätzen in hohen Ausbeuten mit einer geringen Menge an Abfallstoffen hergestellt werden kann. Diese Aufgabe wurde durch die vorliegende Erfindung gelöst.

Gegenstand der Erfindung ist demgemäß ein Verfahren zur Herstellung von Diacetonketogulonsäure durch elektrochemische Oxidation oder Luftoxidation von Diacetonsorbose, das dadurch gekennzeichnet ist, daß die zu etwa 30-95 % teiloxidierte Reaktionslösung durch Elektrodialyse aufgetrennt wird, wobei die reine Diacetonketogulonsäure gewonnen wird und die nichtoxidierte Diacetonsorbose in die Oxidation zurückgeführt wird.

Der Vorteil dieser Verfahrensweise ist, daß die Oxidation im optimalen Bereich mit relativ hohen Konzentrationen des Ausgangsmaterials abläuft und die aufwendige Restoxidation bis zu 100 %igem Umsatz des Ausgangsmaterials unterbleiben kann, obwohl natürlich durch die Rückführung der nichtoxidierten Diacetonsorbose letzten Endes eine 100 %ige Umsetzung erfolgt. In einer bevorzugten Ausgestaltung des Verfahrens wird sowohl die Oxidation als auch die Abtrennung der Diacetonketogulonsäure kontinuierlich durchgeführt.

Die zunächst durchgeführte Oxidation der Diacetonsorbose erfolgt nach an sich bekannten Verfahren. Die durch Edelmetalle katalysierte Luftoxidation ist z. B. beschrieben in der DE-PS 935 968, der HU-PS 162 772 und der DOS 21 23 621 und die elektrochemische Oxidation z. B. in Tetrahedron 28, 37-42 (1972), Elektrochimia 6, 897-909 (1972), der DOS 16 68 203 oder der DOS 25 05 911.

Nach diesen Verfahren werden etwa 5 bis 15 %ige Lösungen von Diacetonsorbose eingesetzt, die mit einer Alkalilauge, vorzugsweise Natronlauge auf einen pH-Wert von etwa 12 bis etwa 14 gebracht werden. Dieser pH-Wert wird in der Regel durch weitere Zugabe von Alkali im Verlauf der unter Bildung von Diacetonketogulonsäure ablaufenden Reaktion konstant gehalten.

Im Gegensatz zu den bekannten Verfahren wird bei dem erfindungsgemäßen Verfahren die Oxidation jedoch nicht bis zu einem maximalen Umsatz durchgeführt, sondern nur bis zu einem Umsatz von etwa 30-95 %, vorzugsweise 50-85 %, des Ausgangsmaterials geführt. Die Oxidationslösung, die etwa zwischen 6 und 65 g Diacetonsorbose, 65 bis 130 g Diacetonketogulonsäure und 10 bis 30 g NaOH pro kg Lösung enthält, wird dann der Dialyse zugeführt.

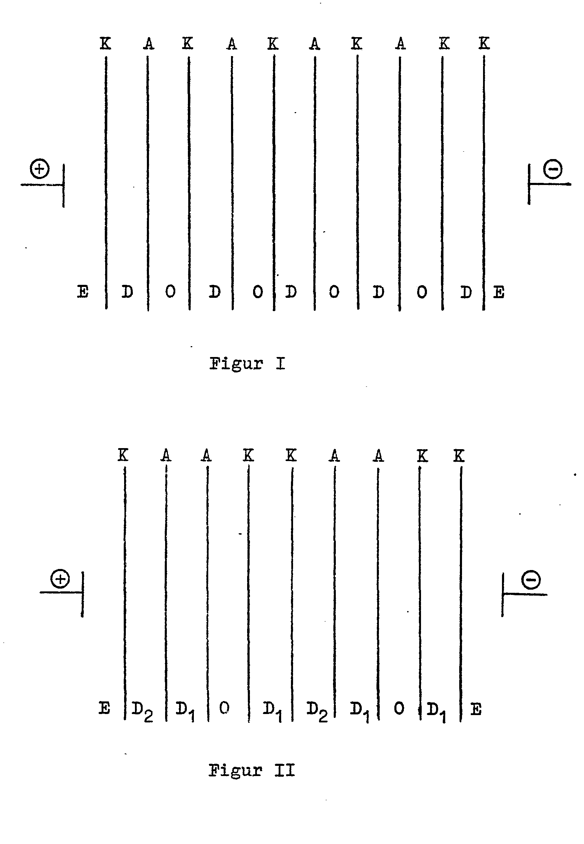

Dazu werden übliche Elektrodialyseanlagen verwendet, bei denen eine Reihe von Kationen- und Anionenaustauschermembranen einzeln oder in Gruppen alternierend angeordnet sind. Typische Membrananordnungen sind in den Fig. 1 und 2 dargestellt. Mit A und K sind darin die Anionen- und Kationenaustauschermembranen bezeichnet. D und O sind die Kammern für Dialysat und die Oxidationslösung bzw. Retentat. Mit E sind die Elektrodenkammern bezeichnet.

Die aus der Oxidation stammende Lösung wird durch die mit o bezeichneten Kammern umgepumpt, die durch jeweils eine Kationen- und Anionenaustauschermembran von den Dialysatkammern D getrennt sind. Bei Anlegen einer elektrischen Gleichspannung an die elektroden wandern elektrisch geladene Teilchen durch die Membranen in die Dialysat-Lösung. Sowohl die Oxidationslösung als auch die Dialysat-Lösung wird im Kreislauf umgepumpt, um die Konzentrationspolarisation an den Membranen möglichst gering zu halten. Die Umwälzgeschwindigkeit ist stark abhängig von Zahl und Geometrie der Kammern, die Umwälzgeschwindigkeit wird jedoch in der Regel so eingestellt, daß Strömungsgeschwindigkeiten längs der Membranen von etwa 0,1 bis etwa 0,3 Meter/Sekunde erreicht werden. In den beiden Elektrodenkammern wird eine elektrodenlösung umgepumpt, die nur dem Zweck dient, daß eine entsprechende Leitfähigkeit sowie der Gas- und Wärmetransport aufrecht erhalten wird. Die Elektrodenlösung kann deshalb z. B. Elektrolyte wie z. B. Natriumsulfat oder NaOH enthalten. Die an die Elektroden anzulegende Gleichspannung hängt ab vom Salzgehalt der Lösungen in den einzelnen Kammersegmenten. In der Regel wird zu Beginn der Dialyse eine Spannung von etwa 0,5 bis etwa 1,5 Volt pro Zelle angelegt, die gegen Ende auf etwa 2 bis etwa 4 Volt pro Zelle ansteigen kann. Die Temperatur der Lösungen ist an sich nicht kritisch, man wird jedoch vorzugsweise, um die Viskosität und Leitfähigkeit der Lösungen vorteilhaft zu beeinflussen, Temperaturen von über Raumtemperatur bis etwa 75 °C wählen.

Als Membranen können alle auf dem Kationen- und Anionenaustauscherprinzip beruhenden Membranen verwendet werden, deren durchlässigkeit ausreichend groß ist um die relativ sperrigen Moleküle der Diacetonketogulonsäure passieren zu lassen. Beispielsweise sind dafür geeignet Membranen auf der Basis von polymeren Ionenaustauschern wie z. B. die Typen Selemion® der Firma Asahi Glass oder die Typen Neosepta® der Firma Tokyama Soda oder ähnliche Membranen der Firmen Ionics und Du Pont.

Bei der Abtrennung der Diacetonketogulonsäure hat sich überraschenderweise gezeigt, daß bei der Elektrodialyse der alkalischen, Diacetonsorbose und Diacetonketogulonsäure enthaltenden Lösung eine Fraktionierung in der Weise auftritt, daß zunächst fast ausschließlich NaOH in das Dialysat übergeht. Erst wenn der NaOH-Gehalt relativ gering ist, geht auch Diacetonketogulonsäure in zunehmendem Maß in das Dialysat über. Dadurch ergibt sich die Möglichkeit, die bei der Oxidation zur Neutralisation der gebildeten Diacetonketogulonsäure zugesetzte Lauge zurückzugewinnen und wieder einzusetzen. Es werden dadurch erhebliche Mengen sowohl an Alkalilauge als auch an Säure, die sonst zur Neutralisation dieser Lauge bei der Diacetonketogulonsäure-Fällung notwendig wären, eingespart und das Abwasser wird von den sonst bei dieser Neutralisation anfallenden Salzen entlastet. Hiermit wird ein guter Betrag zum Umweltschutz geleistet.

Eine solche fraktionierte Dialyse kann entweder Diskontinuierlich geführt werden oder aber kontinuierlich in zumindest zwei hintereinander geschalteten Dialysiervorrichtungen.

Die in der ersten Dialysestufe als Dialysatgewonnene Natronlauge kann unmittelbar in die Oxidationsstufe zurückgeführt werden. Das Retentat der ersten Dialysestufe, das nur noch geringe Mengen, z. B. etwa 0-2 Gew.%, NaOH enthält, aber praktisch die gesamte Menge an Diacetonsorbose und Diacetonketogulonsäure, wird der zweiten dialysestufe zugeführt. Als Dialysat der zweiten Stufe fällt eine Lösung an, die die restliche Natronlauge und die Hauptmenge Diacetonketogulonsäure enthält sowie etwas Diacetonsorbose, die allein aufgrund ihres osmotischen Druckes in geringen Mengen in das Dialysat diffundiert.

Das Retentat, dessen pH-Wert gegen Ende der Dialyse durch Zugabe von NaOH bei einem pH-Wert von etwa 7-8 gehalten wird, enthält bis auf geringe, in das Dialysat übergegangene Verluste die gesamte Menge an Diacetonsorbose und daneben ggf. noch eine Restkonzentration an Diacetonketogulonsäure.

Es wurde bereits erwähnt, daß es vorteilhaft sein kann, die Diacetonketogulonsäure nicht vollständig aus der Oxidationslösung abzutrennen. Der Grund dafür ist, daß der Wirkungsgrad der Dialyse in Bezug auf Diacetonketogulonsäure mit sinkender Diacetonketogulonsäurekonzentration immer ungünstiger wird. Die Dialyse wird deshalb vorzugsweise bei einem Restgehalt der Oxidationslösung an Diacetonketogulonsäure von etwa 1 bis etwa 25 % der Ausgangsmenge abgebrochen. Dies bedeutet keinen Verlust an Diacetonketogulonsäure, da das Retentat ja zunächst der Oxidation und danach erneut der Dialyse zugeführt wird.

Das Dialysat der zweiten Dialysestufe wird der Fällung der Diacetonketogulonsäure zugeführt, die in üblicher Weise durch Ansäuern mit Salzsäure gefällt, isoliert und zu Vitamin C weiterverarbeitet werden kann.

Durch die Erfindung steht somit ein Verfahren zur Verfügung, das es gestattet, die als Zwischenprodukt bei der Vitamin C-Herstellung besonders wertvolle Diacetonketogulonsäure unter sehr geringem Einsatz an Reagenzien und dementsprechend geringen Abfallproblemen durch praktisch vollständigen Umsatz aus Diacetonsorbose zu gewinnen.

In den nachfolgenden Beispielen werden Selemion-Membranen des Typs CMV und AMV der Firma Asahi-Glass verwendet. Mit gleich gutem Erfolg können jedoch auch z. B. die Membrantypen ASV von Asahi-Glass oder die Typen 103 PZL 183, 61 AZL 183, CR 61 MZL 183, CR 61 CYL 183 und 103-QZL-219 der Firma Jonics, Inc sowie andere Membranen wie z. B. die typen Neosepta CL-25T. CH-45T, AV-4T, AF-4t oder AVS-4T der Firma Tokyama Soda oder die Typen Nafion® 152 E, 214, 315 und 427 der Firma Du Pont verwendet werden.

Beispiel 1

In einer Membrananordnung entsprechend Fig. 1 werden 71,6 kg einer alkalischen, zu etwa 63 % elektrochemisch teiloxidierten Diacetonsorboselösung, bestehend aus 1,876 kg NaOH, 3,44 kg Diacetonsorbose und 6,1 kg Diacetonketogulonsäure durch die Oxidationslösungskammern O im Kreislauf gepumpt. Im separaten Kreislauf der Dialysatkammern D wird eine Mischung von 25 kg vollentsalztem Wasser und 400 g 32 Gew.-%iger Natronlauge ebenfalls im Kreislauf geführt. Durch die Elektrodenkammern E wird als dritter Kreislauf eine 10 %ige Natriumsulfatlösung gepumpt.

Nach 4 Stunden Betriebszeit bei einer Gleichstromspannung von 13,5 Volt und, zu Beginn der Dialyse, einem Strom von 40 Ampere, werden 28.7 kg Dialysatlösung I mit einem NaOH-Gehalt von 1,09 kg (51,2% der eingesetzten Menge) abgelassen und durch eine Mischung von 25 kg vollentsalztem Wasser mit 100 g 32 Gew.%iger Natronlauge ersetzt. Nach weiterer Elektrodialyse bei bis zum Ende auf 30 Volt ansteigender Spannung und auf 5,6 Ampere abnehmendem Strom werden 50,8 kg Oxidationslösung mit einem Gehalt von 3,3 kg Diacetonsorbose (96 % der eingesetzten Menge) und 0,55 kg Diacetonketogulonsäure (9,0 % der eingesetzten Menge) abgelassen.

Als Dialysat II werden 43,5 kg Lösung erhalten mit einem Gehalt an Diacetonketogulonsäure von 5,524 kg (90,7 % der eingesetzten Menge), 0,087 kg Diacetonsorbose (2,5 % der eingesetzten Menge) und 0,864 kg NaOH (46,0 % der eingesetzten Menge).

**Beispiel 2**

Eine zu 55,5 % elektrochemisch teiloxidierte Diacetonsorboselösung mit einem Gehalt von 1,44 kg Natronlauge, 2,8 kg Diacetonsorbose und 3,5 kg Diacetonketogulonsäure werden entsprechend Beispiel 1 dialysiert. Es werden die folgenden Ausbeuten erhalten :

Dialysat I : 1,30 kg NaOH (90,2 % der eingesetzten Menge) Dialysat II : 0,36 kg Diacetonsorbose (12,86 % der eingesetzten Menge) 2,96 kg Diacetonketogulonsäure (84,6 % der eingesetzten Menge) und 0,116 kg NaOH (8,05 % der eingesetzten Menge).

Retentat : 2,4 kg Diacetonsorbose (85,7 % der eingesetzten Menge) und 0,5 kg Diacetonketogulonsäure (14,3 % der eingesetzten Menge).

**Beispiel 3**

44 kg einer zu 85,7 % elektrochemisch teiloxidierten Diacetonsorboselösung mit einem Gehalt von 0,88 kg Diacetonsorbose, 5,28 kg Diacetonketogulonsäure und 0,792 kg NaOH werden wie in Beispiel 1 beschrieben elektrodialysiert.

Nach 4 Stunden bei 13 Volt Spannung und anfangs 40 Ampere Strom werden 29,3 kg Dialysat I mit einem Gehalt von 0,015 kg Diacetonsorbose (1,7 % der eingesetzten Menge), 0,67 kg Diacetonketogulonsäure (12,7 % der eingesetzten Menge) und 0,946 kg NaOH (99,62 % der eingesetzten Menge) abgenommen und durch 25 kg vollentsalztem Wasser ersetzt. Nach weiteren 11 Stunden bei einer Spannung zwischen 13 und 18,6 Volt und einem Strom von 9-11 Ampere wird die Elektrodialyse beendet und folgende Lösungen erhalten :

32,5 kg Retentat mit einem Gehalt von 0,81 kg Diacetonsorbose (92 % der eingesetzten Menge) und 1,33 kg Diacetonketogulonsäure (25,2 % der eingesetzten Menge).

35,3 kg Dialysat II mit einem Gehalt von 0,06 kg Diacetonsorbose (6,81 % der eingesetzten Menge) und 3,21 kg Diacetonketogulonsäure (60,8 % der eingesetzten Menge).

**Beispiel 4**

In einer Elektrodialysezelle mit 40 cm² Gesamtmembranfläche werden über 6,25 Stunden 250 ml einer Lösung, die 5 g Diacetonsorbose, 24,8 g Diacetonketogulonsäure und 4,35 g NaOH enthält, dialysiert, wobei die anfängliche Stromdichte von 260 A/m² auf 175 A/m² abfällt. Der pH-Wert der Lösung wird durch Zugabe von NaOH ständig bei etwa 13 gehalten. Man erhält 301 g Dialysat I, das 3,7 g NaOH (85 % der eingesetzten Menge), 0,1 g Diacetonsorbose (2 % der eingesetzten Menge) und 0,7 g Diacetonketogulonsäure (2,8 % der eingesetzten Menge) enthält.

In einer zweiten Dialysestufe mit einer von 175 A/m² auf 110 A/m² fallenden Stromdichte erhält man 323 g Dialysat II, das 0,13 g Diacetonsorbose (2,6 % der eingesetzten Menge) und 16 g Diacetonketogulonsäure (64,5 % der eingesetzten Menge) enthält.

Wiederholt man mit dem Dialysat II die elektrodialytische Abtrennung der Diacetonketogulonsäure in der gleichen Zelle mit einer von 80 A/m² auf 30 A/m² fallenden Stromdichte, erhält man ein Dialysat III, das 0,019 g Diacetonsorbose (0,38 % der eingesetzten Menge) und 12,16 g Diacetonketogulonsäure (49 % der eingesetzten Menge) enthält.

Die durch die mehrstufige Dialyse in einer Dialyseanordnung entsprechend Figur I erreichte Abreicherung der Diacetonsorbose aus dem die Diacetonketogulonsäure enthaltenden Dialysat durch erneutes Einsetzen des Dialysats als Originallösung und Abnahme eines abgereicherten Dialysats kann auch in einem einstufigen Verfahren erreicht werden. Dazu wird eine Dialyseanordnung entsprechend Figur II verwendet. Dabei wird das Dialysat $D_2$, das wegen des zweimaligen Durchgangs durch die Austauschmembranen stark abgereichert an der durch Diffusion mitübergehenden Diacetonsorbose ist, als Dialysat entnommen.

**Beispiel 5**

In einer Elektrodialyse-Zelle mit 175 cm² Membranfläche werden in 15 Stunden 0,5 l einer Lösung, die 16,5 g Diacetonsorbose, 51,5 g Diacetonketogulonsäure und 10,6 g NaOH enthält, getrennt. Es werden erhalten :

Im Dialysat I 8,2 g NaOH (77,4 % der eingesetzten Menge) mit wenig Diacetonsorbose und Diacetonketogulonsäure.

Im Dialysat II 2,0 g NaOH (18,9 % der eingesetzten Menge), 0,5 g Diacetonsorbose (3,0 % der eingesetzten Menge) und 45,0 g Diacetonketogulonsäure (87,0 % der eingesetzten Menge).

Das Retentat mit 15,5 g Diacetonsorbose (93,9 % der eingesetzten Menge) und 6,5 g Diacetonketogulonsäure (12,6 % der eingesetzten Menge).

**Ansprüche**

1. Verfahren zur Herstellung von Diacetonketogulonsäure durch elektrochemische Oxidation oder Luftoxidation von Diacetonsorbose, dadurch gekennzeichnet, daß die zu etwa 30-95 % teiloxidierte Reaktionslösung durch Elektrodialyse aufgetrennt wird, wobei die reine Diacetonketogulonsäure abgetrennt und die nichtoxidierte Diacetonsorbose in die Oxidation zurückgeführt wird.

2. Verfahren nach Anspruch 1, dadurch gekennzeichnet, daß sowohl die Oxidation als auch die Auftrennung kontinuierlich erfolgen.

3. Verfahren nach einem der Ansprüche 1 oder 2, dadurch gekennzeichnet, daß die gebildete Diacetonketogulonsäure nur teilweise abgetrennt wird und der nicht abgetrennte Rest zusammen mit der Diacetonsorbose in die Oxidation zurückgeführt wird.

4. Verfahren nach einem der Ansprüche 1 bis 3, dadurch gekennzeichnet, daß Elektrodialyseanlagen verwendet werden, bei denen eine Reihe von Kationen- und Anionenaustauschermembranen einzeln oder in Gruppen alternierend angeordnet sind.

5. Verfahren nach einem der Ansprüche 1 bis 4, dadurch gekennzeichnet, daß die bei der Elektrodialyse abgetrennte Natronlauge in den Oxidationsprozeß zurückgeführt wird.

6. Verwendung der nach einem der Ansprüche 1 bis 5 hergestellten Diacetonketogulonsäure zur Herstellung von Vitamin C.

## Claims

1. Process for the preparation of diacetoneketogulonic acid by the electrochemical oxidation or atmospheric oxidation of diacetonesorbose, characterised in that the reaction solution, partially oxidised to the extent of about 30-95 %, is separated by electrodialysis, the pure diacetoneketogulonic acid being separated off and the unoxidised diacetonesorbose being recycled to the oxidation.

2. Process according to Claim 1, characterised in that both the oxidation and the separation are carried out continuously.

3. Process according to one of Claims 1 or 2, characterised in that only part of the diacetoneketogulonic acid formed is separated off and the remainder, which is not separated off, is recycled to the oxidation together with the diacetonesorbose.

4. Process according to one of Claims 1 to 3, characterised in that electrodialysis units are used in which a series of cation- and anion-exchange membranes are arranged alternately, either individually or in groups.

5. Process according to one of Claims 1 to 4, characterised in that the sodium hydroxide solution separated off in the electrodialysis is recycled to the oxidation process.

6. Use of the diacetone-ketogulonic acid prepared according to one of Claims 1 to 5, for the preparation of vitamin C.

## Revendications

1. Procédé de préparation d'acide diacétone-cétogulonique par oxydation électrochimique ou oxydation à l'air de diacétone-sorbose, caractérisé en ce qu'on sépare par électrodialyse la solution réactionnelle partiellement oxydée à environ 30-95 %, en ce que l'on retire l'acide diacétone-cétogulonique pur et en ce qu'on réintroduit dans l'oxydation le diacétone-sorbose non oxydé.

2. Procédé selon la revendication 1, caractérisé en ce que tant l'oxydation que la séparation s'effectuent en continu.

3. Procédé selon l'une des revendications 1 ou 2, caractérisé en ce que l'acide diacétone-cétogulonique formé n'est que partiellement séparé et en ce que le résidu non séparé est réintroduit avec le diacétone-sorbose dans l'oxydation.

4. Procédé selon l'une des revendications 1 à 3, caractérisé en ce qu'on utilise des installations d'électrodialyse dans lesquelles est disposée une série de membranes échangeuses de cations et d'anions isolément ou en groupes de façon alternée.

5. Procédé selon l'une des revendications 1 à 4, caractérisé en ce que la lessive de soude séparée dans l'électro-dialyse est réintroduite dans le procédé d'oxydation.

6. Application de l'acide diacétone-cétogulonique préparé selon l'une des revendications 1-5 à la préparation de la vitamine C.

K   A   K   A   K   A   K   A   K   K

⊕

⊖

E   D   O   D   O   D   O   D   O   D   E

Figur I

K   A   A   K   K   A   A   K   K

⊕

⊖

E   $D_2$   $D_1$   O   $D_1$   $D_2$   $D_1$   O   $D_1$   E

Figur II

1